# EUROPEAN PATENT SPECIFICATION

(11) **EP 3 558 483 B1**
(45) Date of publication and mention of the grant of the patent: **04.11.2020**
(21) Application number: 17829036.7
(22) Date of filing: 19.12.2017
(51) Int. Cl.: B01D 3/00, C07C 7/04, C07C 7/08, C07C 11/02

(54) **METHOD OF SEPARATING LINEAR ALPHA OLEFINS**
VERFAHREN ZUR TRENNUNG VON LINEAREN ALPHA-OLEFINEN
PROCÉDÉ DE SÉPARATION D'ALPHA-OLÉFINES LINÉAIRES

(30) Priority: 20.12.2016 US 201662436499 P
(43) Date of publication of application: 30.10.2019
(73) Proprietor: SABIC Global Technologies B.V., 4612 PX Bergen op Zoom (NL)
(72) Inventor: ALHAZMI, Khalid Mohammed, Riyadh 11551 (SA); RALLAPALLI, Jagan, Riyadh 11551 (SA)
(74) Representative: Balder IP Law, S.L.
(86) International application number: PCT/IB2017/058170
(87) International publication number: WO 2018/116181

(56) References cited:
- EP-A1- 2 738 151
- WO-A1-2010/056257
- US-A1- 2007 185 362

## Description

### BACKGROUND

Linear olefins are one of the most useful classes of hydrocarbons used as raw materials in the petrochemical industry. Among these linear alpha-olefins, unbranched olefins whose double bond is located at a terminus of the chain, form an important subclass. Linear alpha olefins can be converted to linear primary alcohols by hydroformylation. Hydroformylation can also be used to prepare aldehydes as the major products which in turn can be oxidized to afford synthetic fatty acids, especially those with an odd carbon number, useful in the production of lubricants. Linear alpha olefins are also used in the most important class of detergents for domestic use, namely the linear alkylbenzene sulfonates, which are prepared by Friedel-Crafts reaction of benzene with linear olefins followed by sulfonation.

Although linear olefins are the product of dehydrogenation of linear alkanes, the major portion of such products consists of the internal olefins. Preparation of alpha olefins is based largely on oligomerization of ethylene, which has a corollary that the alpha-olefins produced have an even number of carbon atoms. Oligomerization processes for ethylene are based mainly on organoaluminum compounds or transition metals as catalysts.

Oligomerization methods for preparing linear alpha-olefins are widely known in the art. These methods are typically carried out in the presence of a catalyst, preferably comprising a zirconium component, such as zirconium tetraisobutyrate, and an aluminum component as activator, such as ethyl aluminum sesquichloride.

Typically, the effluent from the reactor used to produce the linear alpha olefins is directed to one or more distillation columns to separate the various fractions of linear alpha olefins. One problem associated with the recovery of various fractions of produced linear alpha olefins includes impurities, such as solvents and catalysts, contaminating the various fractions. For example, under certain conditions, such as during a plant startup or feed flow interruptions, the amount of impurities in the C4 and C6 streams may reach up to 10,000 parts per million. As a result, the isolated fractions are off-specification (i.e., do not meet market demands of purity) and cannot be used without further purification.

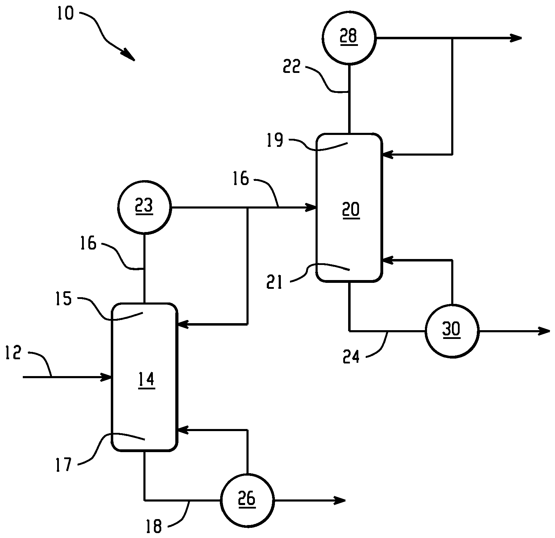

In the production of linear alpha olefins, distillation columns are used to separate light hydrocarbons, for example, ethylene and butane, from heavier hydrocarbons. These separation processes present many engineering challenges. For example, a stream of linear alpha olefins produced by the oligomerization of ethylene can comprise C4-C20 straight chain hydrocarbon molecules. These linear alkenes can serve as building blocks in a wide range of industrial applications. For example, linear alkenes can serve as co-monomers, plasticizers, lubricants and stabilizers. Accordingly, the separation of these olefins from an oligomerization reactor product stream remains a commercially important goal. Furthermore, it is important that these products are of a high purity level that can meet industry quality standards. EP 2738151 discloses in Figure 1 a process for the separation of a mixture of alpha-olefins.

Starting with a stream of light hydrocarbons such as butane, linear alkenes are often separated using a series of distillation columns. The narrow range of boiling points and relative volatilities of the components in the stream make separation by conventional distillation difficult and energy intensive. For example, separation from this stream is often accomplished using a direct sequence of two distillation columns in series. Each of the distillation columns in this direct sequence comprises both a reboiler and a condenser. As a result, the direct sequence consumes a high and undesirable amount of energy, for example, the columns can demand a high reboiler duty and a high condenser duty.

Thus, there is a need for an efficient method of separating linear alpha olefins that can significantly reduce energy consumption while maintaining product flow rate and product purity.

### SUMMARY

Disclosed, in various embodiments, are methods of separating linear alpha olefins.

A method of separating linear alpha olefins includes: passing a feed stream comprising linear alpha olefins through a first column; distributing a C10- fraction to a top portion of the first column; distributing a C12+ fraction to a bottom portion of the first column; withdrawing the C10- fraction from the top portion of the first column; passing the C10- fraction through a second column; distributing a C8 fraction to a top portion of the second column; and distributing a C10 fraction to a bottom portion of the second column; wherein any fraction withdrawn from the top portion of the first column is further separated into greater than or equal to two fractions.

A method of separating linear alpha olefins includes: passing a feed stream comprising C4 to C20 linear alpha olefins through a first column, wherein the source of the feed stream is the product of an ethylene oligomerization process; distributing a C10- fraction to a top portion of the first column; distributing a C12+ fraction to a bottom portion of the first column; withdrawing the C10- fraction from the top portion of the first column; passing the C10- fraction through a second column; distributing a C8 fraction to a top portion of the second column, wherein the C8 fraction comprises greater than or equal to 99.9% octene; and distributing a C10 fraction to a bottom portion of the second column, wherein the C10 fraction comprises greater than or equal to 99.9% decene; wherein a total reboiler duty is less than or equal to 1350 kilowatts, wherein a total condenser duty is less than or equal to 1300 kilowatts, and wherein a total amount of energy consumed by the method is reduced by greater than or equal to 20.5% as compared to a direct method of linear alpha olefin separation, wherein any fraction withdrawn from the top portion of the first column is further separated into greater than or equal to two fractions.

These and other features and characteristics are more particularly described below.

### BRIEF DESCRIPTION OF THE DRAWINGS

The following is a brief description of the drawings wherein like elements are numbered alike and which are presented for the purposes of illustrating the exemplary embodiments disclosed herein and not for the purposes of limiting the same.

FIG. 1 is a schematic diagram representing a distillation column scheme in a method of separating linear alpha olefins.

### DETAILED DESCRIPTION

Disclosed herein is a method that can efficiently separate linear alpha olefins. The method can significantly reduce energy consumption, while maintaining product flow rate and product purity. For example, the method disclosed herein can reduce energy consumption by greater than or equal to 20%. This is true for both the total condenser duty and the total reboiler duty. A high product flow rate and high product purity (e.g., 99.9 weight %) can also be maintained by the present method even at this lower energy level. The reduction in energy seen in the present method can also result in a 20% or greater reduction in total operating costs. Furthermore, it is noted that modification of the sequence does not require the purchase of new equipment.

Linear alpha olefin fractions generally are separated using a direct sequence of two distillation columns in series, wherein the lightest component is withdrawn as a top product from each column. The bottom fraction withdrawn from the bottom portion of the first column is generally further separated into two fractions. In the disclosed method, fractions are separated using a modified indirect sequence of two distillation columns in series, wherein the heaviest component is withdrawn as a bottom product from each column. The top fraction withdrawn from the top portion of the first column is further separated into two fractions. For example, the method disclosed herein can include withdrawing a C10-fraction from a top portion of a first column, passing the C10- fraction through a second column, distributing a C8 fraction to a top portion of the second column and distributing a C10 fraction to a bottom portion of the second column. With these modifications, it was surprisingly discovered that the desired purity levels can be achieved and energy consumption can also be reduced by greater than or equal to 20%.

The method can include passing a feed stream through a column, for example, a first distillation column. The feed stream can comprise hydrocarbons, for example, C4-C20 linear alpha olefins. For example, the source of the feed stream can be the product of a linear alpha olefin production process, for example, ethylene oligomerization. The feed stream can be a product of a C4/C6 separation column. The feed stream can also comprise a solvent, for example, toluene, as well as spent catalyst particles and catalyst deactivating agents.

"Ethylene oligomerization" combines ethylene molecules to produce linear alpha-olefins of various chain lengths with an even number of carbon atoms. This approach results in a distribution of alpha-olefins. Oligomerization of ethylene can produce 1-hexene.

1-Hexene is commonly manufactured by two general routes: (i) full-range processes via the oligomerization of ethylene and (ii) on-purpose technology. A minor route to 1-hexene, used commercially on smaller scales, is the dehydration of hexanol. Prior to the 1970s, 1-hexene was also manufactured by the thermal cracking of waxes. Linear internal hexenes were manufactured by chlorination/dehydrochlorination of linear paraffins.

Fischer-Tropsch synthesis to make fuels from synthesis gas derived from coal can recover 1-hexene from the aforementioned fuel streams, where the initial 1-hexene concentration cut can be 60% in a narrow distillation, with the remainder being vinylidenes, linear and branched internal olefins, linear and branched paraffins, alcohols, aldehydes, carboxylic acids, and aromatic compounds. The trimerization of ethylene by homogeneous catalysts has been demonstrated.

There are a wide range of applications for linear alpha olefins. The lower carbon numbers, 1-butene, 1-hexene and 1-octene can be used as comonomers in the production of polyethylene. High density polyethylene (HDPE) and linear low density polyethylene (LLDPE) can use approximately 2-4% and 8-10% of comonomers, respectively.

Another use of C₄-C₈ linear alpha olefins can be for production of linear aldehyde via oxo synthesis (hydroformylation) for later production of short-chain fatty acid, a carboxylic acid, by oxidation of an intermediate aldehyde, or linear alcohols for plasticizer application by hydrogenation of the aldehyde.

An application of 1-decene is in making polyalphaolefin synthetic lubricant base stock (PAO) and to make surfactants in a blend with higher linear alpha olefins.

C₁₀-C₁₄ linear alpha olefins can be used in making surfactants for aqueous detergent formulations. These carbon numbers can be reacted with benzene to make linear alkyl benzene (LAB), which can be further sulfonated to linear alkyl benzene sulfonate (LABS), a popular relatively low cost surfactant for household and industrial detergent applications.

Although some C14 alpha olefin can be sold into aqueous detergent applications, C₁₄ has other applications such as being converted into chloroparaffins. A recent application of C₁₄ is as on-land drilling fluid base stock, replacing diesel or kerosene in that application. Although C₁₄ is more expensive than middle distillates, it has a significant advantage environmentally, being much more biodegradable and in handling the material, being much less irritating to skin and less toxic.

C₁₆ - C₁₈ linear olefins find their primary application as the hydrophobes in oil-soluble surfactants and as lubricating fluids themselves. C₁₆ - C₁₈ alpha or internal olefins are used as synthetic drilling fluid base for high value, primarily off-shore synthetic drilling fluids. The preferred materials for the synthetic drilling fluid application are linear internal olefins, which are primarily made by isomerizing linear alpha-olefins to an internal position. The higher internal olefins appear to form a more lubricious layer at the metal surface and are recognized as a better lubricant. Another application for C₁₆ - C₁₈ olefins is in paper sizing. Linear alpha olefins are, once again, isomerized into linear internal olefins and then reacted with maleic anhydride to make an alkyl succinic anhydride (ASA), a popular paper sizing chemical.

C₂₀ - C₃₀ linear alpha olefins production capacity can be 5-10% of the total production of a linear alpha olefin plant. These are used in a number of reactive and nonreactive applications, including as feedstocks to make heavy linear alkyl benzene (LAB) and low molecular weight polymers used to enhance properties of waxes.

The use of 1-hexene can be as a comonomer in production of polyethylene. High-density polyethylene (HDPE) and linear low-density polyethylene (LLDPE) use approximately 2-4% and 8-10% of comonomers, respectively.

Another use of 1-hexene is the production of the linear aldehyde heptanal via hydroformylation (oxo synthesis). Heptanal can be converted to the short-chain fatty acid heptanoic acid or the alcohol heptanol.

A pressure within the first column can be 0 kiloPascals to 200 kiloPascals, for example, 20 kiloPascals to 150 kiloPascals, for example, 125 kiloPascals. A temperature within a top portion of the first column can be 100°C to 200°C, for example, 120°C to 160°C, for example, 142°C. A temperature within a bottom portion of the first column can be 200°C to 300°C, for example, 220°C to 260°C, for example, 247°C.

In the present method, linear alpha olefin fractions can be separated using a modified indirect sequence of two distillation columns in series, wherein the heaviest component is withdrawn as a bottom product from each column. For example, a C12+ fraction can be distributed to and withdrawn from a bottom portion of the first column. The C12+ fraction can be passed through a reboiler for the first column. The reboiler can heat and partially vaporize at least a portion of the C12+ fraction. For example, the reboiler can include a spiral heat exchanger and/or a plate heat exchanger. The reboiler can utilize a stream of heating fluid as a heating means. A portion of the C12+ fraction can be recycled back to the bottom portion of the first column. The C12+ fraction can be passed to further downstream units for additional processing. For example, the C12+ fraction can be passed through additional separation columns.

A C10- fraction can be distributed to and withdrawn from a top portion of the first column. The C10- fraction can be passed through a condenser for the first column. The condenser can cool and partially condense at least a portion of the C10- fraction. For example, the condenser can include a spiral heat exchanger and/or a plate heat exchanger. The condenser can utilize a stream of coolant fluid as a cooling means. A portion of the C10-fraction can be recycled back to the top portion of the first column.

In the present method, linear alpha olefin fractions can be separated using a modified indirect sequence of two distillation columns in series, wherein the top fraction withdrawn from the top portion of the first column is further separated into two fractions. For example, the C10- fraction can be passed through a second distillation column.

A pressure within the second column can be 0 kiloPascals to 200 kiloPascals, for example, 20 kiloPascals to 150 kiloPascals, for example, 125 kiloPascals. A temperature within a top portion of the second column can be 100°C to 200°C, for example, 120°C to 160°C, for example, 129°C. A temperature within a bottom portion of the second column can be 100°C to 200°C, for example, 160°C to 190°C, for example, 179°C.

A C10 fraction can be distributed to and withdrawn from a bottom portion of the second column. The C10 fraction can be passed through a reboiler for the second column. A portion of the C10 fraction can be recycled back to the bottom portion of the second column. The C10 fraction can comprise greater than or equal to 99 weight % (wt.%) decene, for example, greater than or equal to 99.9 wt.% decene. A flow rate for the C10 fraction can be greater than or equal to 2000 kilograms per hour (kg/hr), for example, greater than or equal to 2200 kg/hr, for example, 2200.349 kg/hr.

A C8 fraction can be distributed to and withdrawn from a top portion of the second column. The C8 fraction can be passed through a condenser for the second column. A portion of the C8 fraction can be recycled back to the top portion of the second column. The C8 fraction can comprise greater than or equal to 99 wt.% octene, for example, greater than or equal to 99.9 wt.% octene. A flow rate for the C8 fraction can be greater than or equal to 3000 kg/hr, for example, greater than or equal to 3100 kg/hr, for example, 3181.667 kg/hr.

A more complete understanding of the components, processes, and apparatuses disclosed herein can be obtained by reference to the accompanying drawings. These figures (also referred to herein as "FIG.") are merely schematic representations based on convenience and the ease of demonstrating the present disclosure, and are, therefore, not intended to indicate relative size and dimensions of the devices or components thereof and/or to define or limit the scope of the exemplary embodiments. Although specific terms are used in the following description for the sake of clarity, these terms are intended to refer only to the particular structure of the embodiments selected for illustration in the drawings, and are not intended to define or limit the scope of the disclosure. In the drawings and the following description below, it is to be understood that like numeric designations refer to components of like function.

Referring now to FIG. 1, this simplified schematic diagram represents a distillation column scheme 10 in a method for separating linear alpha olefins. The method can include passing a feed stream 12 through a first distillation column 14. For example, the feed stream 12 can comprise hydrocarbons, for example, C4-C20 linear alpha olefins. The source of the feed stream 12 can be the product of a linear alpha olefin production process, for example, ethylene oligomerization.

A C12+ fraction 18 can be distributed to and withdrawn from a bottom portion 17 of the first column 14. The C12+ fraction 18 can be passed through a reboiler 26 for the first column 14. A portion of the C12+ fraction 18 can be recycled back to the bottom portion 17 of the first column 14.

A C10- fraction 16 can be distributed to and withdrawn from a top portion 15 of the first column 14. The C10- fraction 16 can be passed through a condenser 23 for the first column 14. A portion of the C10- fraction 16 can be recycled back to the top portion 15 of the first column 14. The C10- fraction can be passed through a second distillation column 20.

A C10 fraction 24 can be distributed to and withdrawn from a bottom portion 21 of the second column 20. The C10 fraction 24 can be passed through a reboiler 30 for the second column 20. A portion of the C10 fraction can be recycled back to the bottom portion 21 of the second column 20.

A C8 fraction 22 can be distributed to and withdrawn from a top portion 19 of the second column 20. The C8 fraction 22 can be passed through a condenser 28 for the second column 20. A portion of the C8 fraction 22 can be recycled back to the top portion 19 of the second column 20.

The following example is merely illustrative of the method of separating linear alpha olefins disclosed herein and is not intended to limit the scope hereof. Unless otherwise stated, the example was based upon simulations.

### EXAMPLES

Simulations are conducted using a steady state process model in ASPEN PLUS (commercial process engineering software). Total condenser duty is reported in kilowatts (kw) as is total reboiler duty. Flow rate is reported in kilograms per hour (kg/hr) and purity is reported in weight percent (wt.%).

### Example 1

A non-modified direct distillation sequence is simulated for the purposes of this example. C8 and C10 fractions are separated from a linear alpha olefin stream using a direct sequence of two distillation columns in series, wherein the lightest component is withdrawn as a top product from each column. The bottom fraction withdrawn from the bottom portion of the first column is further separated into two fractions. The results are presented in Table 1. The total reboiler duty is calculated as the sum of the reboiler duty for both distillation columns. The total condenser duty is the sum of the condenser duty for both columns.

### Example 2

A modified distillation sequence in accordance with the method disclosed herein is simulated for the purposes of this example. C8 and 10 fractions are separated from a linear alpha olefin stream using a modified indirect sequence of two distillation columns in series, as depicted in FIG. 1, wherein the heaviest component is withdrawn as a bottom product from each column. The top fraction withdrawn from the top portion of the first column is further separated into two fractions. The results are presented in Table 1. The total reboiler duty is calculated as the sum of the reboiler duty for both distillation columns. The total condenser duty is the sum of the condenser duty for both columns.

| **Table 1: Comparison of Distillation Results** | | |
|---|---|---|
| | Example 1 | Example 2 |
| Total Condenser Duty (kw) | -1627.36 | -1294.02 |
| Total Reboiler Duty (kw) | 1645.199 | 1311.858 |
| C8 Flow Rate (kg/hr) | 3181.667 | 3181.667 |
| C8 Purity (wt.%) | 0.999 | 0.999 |
| C10 Flow Rate (kg/hr) | 2200.349 | 2200.349 |
| C10 Purity (wt.%) | 0.999 | 0.999 |

As can be seen from Table 1, the present method (Example 2), as depicted in FIG 1, achieves a greater than or equal to 20% energy reduction when compared to the non-modified sequence of Example 1. This is true for both the total condenser duty (20.5% reduction) and the total reboiler duty (20.3% reduction). Table 1 also shows that a high product flow rate and high product purity (99.9%) can be maintained even at this lower energy level. This is true for both the C8 and C10 fractions. The reduction in energy seen in the present method can also result in a 20% or greater reduction in total operating costs. Modification of the sequence from direct to indirect does not require the purchase of new equipment.

## Claims

1. A method of separating linear alpha olefins, comprising:
passing a feed stream comprising linear alpha olefins through a first column;
distributing a C10- fraction to a top portion of the first column;
distributing a C12+ fraction to a bottom portion of the first column;
withdrawing the C10- fraction from the top portion of the first column;
passing the C10- fraction through a second column;
distributing a C8 fraction to a top portion of the second column; and
distributing a C10 fraction to a bottom portion of the second column;
wherein any fraction withdrawn from the top portion of the first column is further separated into greater than or equal to two fractions.

2. The method of Claim 1, wherein the source of the feed stream is the product of an ethylene oligomerization process.

3. The method of any of the preceding claims, wherein the source of the feed stream is the product of a C4/C6 separation column.

4. The method of any of the preceding claims, wherein the feed stream comprises C4 to C20 linear alpha olefins.

5. The method of any of the preceding claims, wherein the C10 fraction comprises decene, preferably greater than or equal to 99% decene, more preferably greater than or equal to 99.9% decene.

6. The method of any of the preceding claims, wherein the C8 fraction comprises octene, preferably greater than or equal to 99% octene, more preferably greater than or equal to 99.9% octene.

7. The method of any of the preceding claims, wherein the first and/or second column comprises a reboiler and/or a condenser.

8. The method of Claim 7, wherein a total reboiler duty is less than or equal to 1350 kilowatts, or wherein a total condenser duty is less than or equal to 1300 kilowatts.

9. The method of Claim 7, wherein a total reboiler duty is reduced by greater than or equal to 20% as compared to a direct method of linear alpha olefin separation.

10. The method of Claim 7, wherein a total condenser duty is reduced by greater than or equal to 20% as compared to a direct method of linear alpha olefin separation.

11. The method of any of the preceding claims, wherein a total amount of energy consumed by the method is reduced by greater than or equal to 20.5% as compared to a direct method of linear alpha olefin separation.

12. The method of any of the preceding claims, wherein the feed stream comprises ethylene.

13. The method of any of the preceding claims, further comprising withdrawing the C12+ fraction from the bottom portion of the first column, withdrawing the C8 fraction from the top portion of the second column, withdrawing the C10 fraction from the bottom portion of the second column, or a combination comprising at least one of the foregoing.

14. The method of any of the preceding claims, further comprising passing the C12+ fraction through a third column.

15. The method of Claim 1, wherein the feed stream comprises linear alpha olefins which are C4 to C20 linear alpha olefins;
wherein the source of the feed stream is the product of an ethylene oligomerization process;
wherein the C8 fraction comprises greater than or equal to 99.9% octene;
wherein the C10 fraction comprises greater than or equal to 99.9% decene;
wherein a total reboiler duty is less than or equal to 1350 kilowatts;
wherein a total condenser duty is less than or equal to 1300 kilowatts; and
wherein a total amount of energy consumed by the method is reduced by greater than or equal to 20.5% as compared to a direct method of linear alpha olefin separation.

## Patentansprüche

1. Verfahren zum Trennen linearer Alpha-Olefine, beinhaltend:
Leiten eines lineare Alpha-Olefine enthaltenden Beschickungsstroms durch eine erste Säule;
Verteilen einer C10- Fraktion auf einen oberen Teil der ersten Säule;
Verteilen einer C12+ Fraktion auf einen unteren Teil der ersten Säule;
Abziehen der C10- Fraktion aus dem oberen Teil der ersten Säule;
Leiten der C10- Fraktion durch eine zweite Säule;
Verteilen einer C8 Fraktion auf einen oberen Abschnitt der zweiten Säule; und
Verteilen einer C10 Fraktion auf einen unteren Teil der zweiten Säule;
wobei jede Fraktion, die aus dem oberen Teil der ersten Säule entnommen wird, weiter in zwei oder mehr Fraktionen aufgeteilt wird.

2. Verfahren nach Anspruch 1, wobei die Quelle des Beschickungsstroms das Produkt eines Ethylen-Oligomerisierungs-Verfahrens ist.

3. Das Verfahren nach einem der vorstehenden Ansprüche, wobei die Quelle des Beschickungsstroms das Produkt einer C4/C6-Trennsäule ist.

4. Verfahren nach einem der vorstehenden Ansprüche, wobei der Beschickungsstrom lineare C4 bis C20 Alpha-Olefine beinhaltet.

5. Verfahren nach einem der vorstehenden Ansprüche, wobei die C10 Fraktion Decen beinhaltet, vorzugsweise gleich 99% Decen oder mehr, besonders bevorzugt gleich 99,9% Decen oder mehr.

6. Verfahren nach einem der vorstehenden Ansprüche, wobei die C8 Fraktion Okten beinhaltet, vorzugsweise gleich 99% Okten oder mehr, weiter bevorzugt gleich 99,9% Okten oder mehr.

7. Verfahren nach einem der vorstehenden Ansprüche, wobei die erste und/oder zweite Säule einen Verdampfer und/oder einen Kondensor beinhaltet.

8. Das Verfahren nach Anspruch 7, bei dem die Gesamtleistung des Verdampfers kleiner oder gleich 1350 Kilowatt ist, oder wobei die Gesamtleistung des Kondensors kleiner oder gleich 1300 Kilowatt ist.

9. Verfahren nach Anspruch 7, bei dem die Gesamtleistung des Verdampfers im Vergleich zu einem direkten Verfahren einer Trennung linearer Alpha-Olefine um 20% oder mehr verringert wird.

10. Verfahren nach Anspruch 7, bei dem die Gesamtleistung des Kondensors im Vergleich zu einem direkten Verfahren einer Trennung linearer Alpha-Olefine um 20% oder mehr reduziert wird.

11. Verfahren nach einem der vorstehenden Ansprüche, bei dem die Gesamtmenge der durch das Verfahren verbrauchten Energie um 20,5% oder mehr im Vergleich zu zu einem direkten Verfahren einer Trennung linearer Alpha-Olefine reduziert wird.

12. Verfahren nach einem der vorstehenden Ansprüche, wobei der Beschickungsstrom Ethylen beinhaltet.

13. Verfahren nach einem der vorhergehenden Ansprüche, ferner umfassend eine Entnahme der C12+ Fraktion aus dem unteren Teil der ersten Säule, eine Entnahme der C8 Fraktion aus dem oberen Teil der zweiten Säule, eine Entnahme der C10 Fraktion aus dem unteren Teil der zweiten Säule, oder eine Kombination beinhaltend mindestens eine der vorstehenden Maßnahmen.

14. Verfahren nach einem der vorstehenden Ansprüche, ferner beinhaltend das Leiten der C12+ Fraktion durch eine dritte Säule.

15. Verfahren nach Anspruch 1, wobei der Beschickungsstrom lineare Alpha-Olefine beinhaltet, die lineare C4 bis C20 Alpha-Olefine sind;
wobei die Quelle des Beschickungsstroms das Produkt eines Ethylen-Oligomerisierungs-Verfahrens ist;
wobei die C8 Fraktion gleich 99,9% Okten oder mehr beinhaltet;
wobei die C10 Fraktion gleich 99,9 % Decen oder mehr beinhaltet;
wobei die Gesamtleistung des Verdampfers gleich 1350 Kilowatt oder weniger ist;
wobei die Gesamtleistung des Kondensors gleich 1300 Kilowatt oder weniger ist; und
wobei die Gesamtmenge der durch das Verfahren verbrauchten Energie um 20,5% oder mehr im Vergleich zu einer direkten Methode einer Trennung linearen Alpha-Olefine reduziert wird.

## Revendications

1. Méthode pour séparer des alpha-oléfines linéaires, comprenant :
le passage d'un courant d'alimentation comprenant des alpha-oléfines linéaires à travers une première colonne ;
la distribution de la fraction en C10- vers une partie de tête de la première colonne ;
la distribution de la fraction en C12+ vers une partie de fond de la première colonne ;
le retrait de la fraction en C10- à partir de la partie de tête de la première colonne ;
le passage de la fraction en C10- à travers une deuxième colonne ;
la distribution de la fraction en C8 vers une partie de tête de la deuxième colonne ; et
la distribution de la fraction en C10 vers une partie de fond de la deuxième colonne ;
dans laquelle toute fraction retirée à partir de la partie de tête de la première colonne est encore séparée en deux fractions ou plus.

2. Méthode selon la revendication 1, dans laquelle la source du courant d'alimentation est le produit d'un procédé d'oligomérisation d'éthylène.

3. Méthode selon l'une quelconque des revendications précédentes, dans laquelle la source du courant d'alimentation est le produit d'une colonne de séparation C4/C6.

4. Méthode selon l'une quelconque des revendications précédentes, dans laquelle le courant d'alimentation comprend des alpha-oléfines linéaires en C4 à C20.

5. Méthode selon l'une quelconque des revendications précédentes, dans laquelle la fraction en C10 comprend du décène, de préférence 99 % ou plus de décène, mieux encore 99,9 % ou plus de décène.

6. Méthode selon l'une quelconque des revendications précédentes, dans laquelle la fraction en C8 comprend de l'octène, de préférence 99 % ou plus d'octène, mieux encore 99,9 % ou plus d'octène.

7. Méthode selon l'une quelconque des revendications précédentes, dans laquelle la première et/ou deuxième colonne comprend un rebouilleur et/ou un condenseur.

8. Méthode selon la revendication 7, dans laquelle la charge totale du rebouilleur est inférieure ou égale à 1350 kilowatts, ou dans laquelle la charge totale du condenseur est inférieure ou égale à 1300 kilowatts.

9. Méthode selon la revendication 7, dans laquelle la charge totale du rebouilleur est réduite de 20 % ou plus en comparaison avec une méthode directe de séparation d'alpha-oléfines linéaires.

10. Méthode selon la revendication 7, dans laquelle la charge totale du condenseur est réduite de 20 % ou plus en comparaison avec une méthode directe de séparation d'alpha-oléfines linéaires.

11. Méthode selon l'une quelconque des revendications précédentes, dans laquelle la quantité totale d'énergie consommée par la méthode est réduite de 20,5 % ou plus en comparaison avec une méthode directe de séparation d'alpha-oléfines linéaires.

12. Méthode selon l'une quelconque des revendications précédentes, dans laquelle le courant d'alimentation comprend de l'éthylène.

13. Méthode selon l'une quelconque des revendications précédentes, comprenant en outre le retrait de la fraction en C12+ à partir de la partie de fond de la première colonne, le retrait de la fraction en C8 à partir de la partie de tête de la deuxième colonne, le retrait de la fraction en C10 à partir de la partie de fond de la deuxième colonne, ou une combinaison comprenant au moins l'un des précédents.

14. Méthode selon l'une quelconque des revendications précédentes, comprenant en outre le passage de la fraction en C12+ à travers une troisième colonne.

15. Méthode selon la revendication 1, dans laquelle le courant d'alimentation comprend des alpha-oléfines linéaires qui sont des alpha-oléfines linéaires en C4 à C20 ;
dans laquelle la source du courant d'alimentation est le produit d'un procédé d'oligomérisation d'éthylène ;
dans laquelle la fraction en C8 comprend 99,9 % ou plus d'octène ;
dans laquelle la fraction en C10 comprend 99,9 % ou plus de décène ;
dans laquelle la charge totale du rebouilleur est inférieure ou égale à 1350 kilowatts ;
dans laquelle la charge totale du condenseur est inférieure ou égale à 1300 kilowatts ; et
dans laquelle la quantité totale d'énergie consommée par la méthode est réduite de 20,5 % ou plus en comparaison avec une méthode directe de séparation d'alpha-oléfines linéaires.
